# EUROPEAN PATENT APPLICATION

(11) **EP 2 915 474 A1**
(43) Date of publication of application: **09.09.2015**
(21) Application number: 13850115.0
(22) Date of filing: 31.10.2013
(51) Int. Cl.: A61B 1/00, A61B 1/04

(54) **EXAMINATION INFORMATION MANAGEMENT DEVICE AND EXAMINATION INFORMATION MANAGEMENT SYSTEM**

(30) Priority: 31.10.2012 JP 2012241119
(71) Applicant: Olympus Corporation, Shibuya-ku Tokyo 151-0072 (JP)
(72) Inventor: TANIGUCHI, Katsuyoshi, Tokyo 151-0072 (JP); NISHIYAMA, Takeshi, Tokyo 151-0072 (JP)
(74) Representative: Gunzelmann, Rainer
(86) International application number: PCT/JP2013/079642
(87) International publication number: WO 2014/069608

(57) **Abstract**

Provided are an examination information management apparatus and the like that can execute downloading even if the downloading and initialization of a receiving device are performed at different institutions, and can appropriately manage the downloaded image data and the examination information in association with each other. An examination information management apparatus 30 includes a data transmitting and receiving unit 32 that performs transmission and reception of data to and from the receiving device, a storage unit 33 that stores the examination information and the image data, a server ID maintaining unit 33c that maintains an identification ID of the storage unit, a registering unit 35b that registers the examination information and the identification ID with the receiving device, an ID determination unit 35c that determines, when the image data, the examination information and the identification ID are received from the receiving device, whether or not the identification ID registered with the receiving device matches the identification ID maintained by the ID maintaining unit, and an examination information generating unit 35a that newly generates the examination information and causes the storage unit to store the image data acquired by the receiving device in association with the newly generated examination information, if the identification IDs are not determined to match each other.

## Description

### Field

The present invention relates to an examination information management apparatus and an examination information management system that manage examination information in examinations using a capsule endoscope which is configured to be introduced into a subject and capture an image of inside of the subject.

### Background

In recent years, examinations using capsule endoscopes that are introduced into subjects and capture images of inside of the subjects have been known in the field of endoscopes. A capsule endoscope is an apparatus having an image capturing function, a wireless communication function, and the like, built in a capsule shaped casing formed in a size introducible into a digestive tract of a subject, and after being swallowed from the mouth of the subject, performs image capturing while moving inside the digestive tract by the peristaltic movement and the like to generate image data. The generated image data are sequentially transmitted wirelessly to outside of the subject from the capsule endoscope.

The image data wirelessly transmitted from the capsule endoscope are received by a receiving device provided outside the subject and accumulated in a built-in memory or a portable memory. After the examination is finished, the image data accumulated in the memory are transferred (downloaded) to an information management apparatus, such as a work station, and subjected to specified image processing. A medical worker performs diagnosis on the subject by observing images generated as above.

When an examination is carried out by using such a capsule endoscope, before the examination, a process of deleting information that has been stored in a memory of a receiving device and registering examination information, such as an examination ID, a patient's ID, and a patient's name, with the receiving device is performed. This process is called initialization of the receiving device and is performed by a work station while the receiving device is in a state of being connected with the work station.

Further, in recent years, establishment of a network system, which manages medical information, has been developed. By transmitting and receiving various medical information including medical images via this network system, for example, various attempts have been made, such as centralized management of the medical images by a single institution, and diagnosis of the medical images by a specialized doctor stationed in an institution different from an institution that has carried out the examination. For example, in Patent Literature 1, a technique is disclosed, in which, in a medical information management network system including a medical information management server and a work station, results of diagnosis are caused to be stored in the medical information management server, based on a server ID of the medical information management server, patient information, and an examination ID given to examination information.

### Citation List

### Patent Literature

Patent Literature 1: International Patent Publication No. 2008/136282

### Summary

### Technical Problem

Conventionally, examination information in a capsule endoscope examination is maintained in a receiving device after initialization of the receiving device but does not remain in a work station. For this reason, even if multiple examinations are performed on the same subject, multiple pieces of examination information on these examinations are independent of one another and are not associated with one another. However, when an examination or medical treatment using an ordinary endoscope is conducted before or after the capsule endoscope examination, or when multiple capsule endoscope examinations are performed on the same subject for a follow-up, it may be desirable that the multiple pieces of examination information should be associated with one another among the examinations different from one another.

In order to have the multiple pieces of examination information on the multiple examinations in association with one another, the pieces of examination information on the examinations and examination status may be managed in an integrated fashion using the work station or a database of a server connected to the work station, without removing the pieces of examination information from the work station even after the initialization of the receiving device.

However, if a method of managing the pieces of examination information in such a capsule endoscope examination is applied to the above-described conventional network system, the following problem may be caused. Specifically, observation of images taken in the capsule endoscope examination for a patient may not be conducted in one institution, but another institution may be requested to conduct the observation. In this case, examination information is registered in a work station at the original institution, and initialization of a receiving device is performed using the examination information. Next, the receiving device is put on the patient, and the capsule endoscope is swallowed by the patient. After that, images are taken and the images are stored in the receiving device. After the images are taken, the receiving device is sent to another institution. In the institution which received the receiving device, the receiving device is connected to a work station to download the images. Next, the observation of the images are conducted, a report is generated, and the report is sent to the original institution. In the original institution which received the report, results of the examination are explained to the patient. As seen above, if an institution in which initialization of a receiving device is performed is different from an institution in which image data is downloaded from the receiving device, it is impossible, during the downloading, to access to a database which acquired the examination information at the time of initialization. This leads to a situation that there is no examination information to be associated with the image data which is to be downloaded. In this situation, the downloading may not be executed depending on the specifications of the receiving device.

The present invention has been made in view of the foregoing, and an object of the invention is to provide an examination information management apparatus and an examination information management system that are able to execute downloading even if the downloading and initialization of the receiving device are performed at different institutions in a capsule endoscope examination for maintaining examination information in both of the work station and the receiving device, and are able to appropriately manage the downloaded image data and the examination information in association with each other. Solution to Problem

To solve the above-mentioned problem and achieve the object, an examination information management apparatus according to the invention manages examination information in an examination using a capsule endoscope for capturing in-vivo images, and a receiving device for receiving image data acquired by the capsule endoscope through the capturing. The examination information management apparatus includes: a data transmitting and receiving unit that performs transmission and reception of data to and from the receiving device; a storage unit that stores the examination information and the image data acquired in the examination; an ID maintaining unit that maintains an identification ID for identifying the storage unit; a registering unit that registers the examination information and the identification ID with the receiving device; a determination unit that determines, when the image data that the receiving device has received from the capsule endoscope, the examination information and the identification ID are received from the receiving device via the data transmitting and receiving unit, whether or not the identification ID registered with the receiving device matches the identification ID maintained by the ID maintaining unit; and an examination information generating unit that newly generates the examination information and causes the storage unit to store the image data acquired by the receiving device in association with the newly generated examination information, if the identification IDs are not determined by the determination unit to match each other.

In the examination information management apparatus, the examination information generating unit associates the identification ID registered with the receiving device with the examination information when the examination information generating unit causes the storage unit to store the newly generated examination information.

The examination information management apparatus further includes a status information updating unit that adds, to the examination information stored in the storage unit, information indicating that the examination information has been registered, when the registering unit registers the examination information and the identification ID with the receiving device. The determination unit determines whether or not the identification ID registered with the receiving device and the identification ID maintained by the ID maintaining unit match each other, when a process of registering, with the receiving device with which first examination information has been registered, second examination information different from the first examination information, is performed. If the identification IDs have been determined by the determination unit to match each other, the status information updating unit acquires the first examination information from the receiving device with which the first examination information has been registered, cancels, from the first examination information stored in the storage unit, information indicating that the first examination information has been registered, and adds, to the second examination information stored in the storage unit, information indicating that the second examination information has been registered.

An examination information management system according to the invention includes the examination information management apparatus, and a receiving device that receives image data generated by the capsule endoscope. Advantageous Effects of Invention

According to the invention, if an identification ID registered with a receiving device and an identification ID maintained by an examination information management apparatus are determined to be not identical to each other, examination information is newly generated, and image data received by the receiving device from a capsule endoscope are caused to be stored in a storage unit in association with the newly generated examination information, and thus, even if downloading and initialization of the receiving device are to be performed at different institutions, the downloading is able to be executed, and the downloaded image data and the examination information are able to be appropriately managed in association with each other.

### Brief Description of Drawings

FIG. 1 is a schematic diagram illustrating a schematic configuration of an examination information management system according to an embodiment of the present invention.
FIG. 2 is a diagram illustrating a schematic configuration of a capsule endoscope and a receiving device illustrated in FIG. 1.
FIG. 3 is a block diagram illustrating a schematic configuration of the examination information management apparatus illustrated in FIG. 1.
FIG. 4 is a block diagram illustrating a schematic configuration of a server illustrated in FIG. 1.
FIG. 5 is a diagram illustrating contents of examination information stored in a database illustrated in FIG. 4.
FIG. 6 is a schematic diagram illustrating an operation form of the examination information management system illustrated in FIG. 1.
FIG. 7 is a flow chart illustrating a series of processes related to an examination using the capsule endoscope illustrated in FIG. 1.
FIG. 8 is a flow chart illustrating an initialization process of the receiving device executed by the examination information management apparatus illustrated in FIG. 3.
FIG. 9 is a schematic diagram illustrating a display example of an examination selection screen.
FIG. 10 is a flow chart illustrating a download process of image data executed by the examination information management apparatus illustrated in FIG. 3.
FIG. 11 is a schematic diagram illustrating a part of an examination information management system according to a first modified example of the embodiment of the present invention.
FIG. 12 is a flow chart illustrating an initialization process of a receiving device executed by the examination information management apparatus illustrated in FIG. 11.
FIG. 13 is a flow chart illustrating an initialization process of a receiving device executed by an examination information management apparatus according to a second modified example.

### Description of Embodiments

Hereinafter, an examination information management apparatus and an examination information management system according to embodiments of the present invention will be described with reference to the drawings. The present invention is not limited by these embodiments. Further, in each drawing, illustration is made by appending the same reference signs to the same portions.

### (Embodiment)

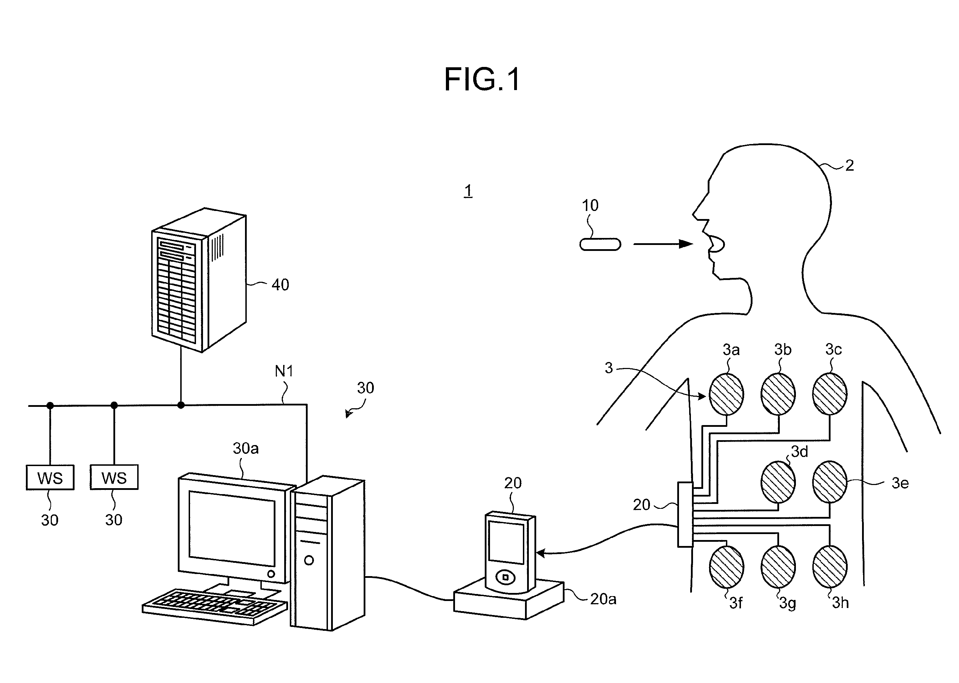

FIG. 1 is a schematic diagram illustrating a schematic configuration of an examination information management system according to an embodiment of the present invention. As illustrated in FIG. 1, an examination information management system 1 according to the embodiment includes: a capsule endoscope 10 that generates image data by being introduced into a subject 2 and capturing an image of inside of the subject 2, and superimpose and transmits the image data on a wireless signal; a receiving device 20 that receives, via a receiving antenna unit 3 attached to the subject 2, the wireless signal transmitted from the capsule endoscope 10; an examination information management apparatus 30 that manages information (examination information) related to an examination performed on the subject 2, and the image data, which are a result of that examination; and a server 40 that is connected to the examination information management apparatus 30 via a network N1 and stores therein the examination information and the image data.

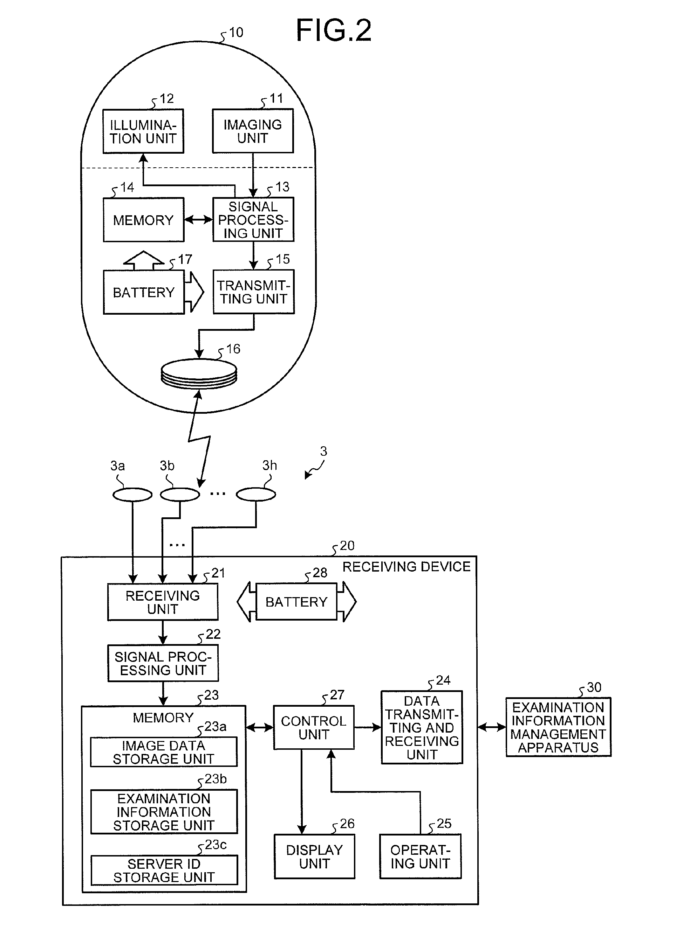

FIG. 2 is a block diagram illustrating a schematic configuration of the capsule endoscope 10 and the receiving device 20.

The capsule endoscope 10 is a device having various parts, such as an imaging element, built in a capsule shaped casing of a size swallowable by the subject 2, and includes: an imaging unit 11 that captures an image of inside of the subject 2; an illumination unit 12 that illuminates the inside of the subject 2; a signal processing unit 13; a memory 14; a transmitting unit 15 and an antenna 16; and a battery 17.

The imaging unit 11 includes, for example: an imaging element such as a CCD or a CMOS, which generates and outputs an imaging signal representing the inside of the subject from an optical image formed on a light receiving surface; and an optical system such as an objective lens, which is arranged on a light receiving surface side of the imaging element.

The illumination unit 12 is realized by a light emitting diode (LED) or the like that emits light towards the inside of the subject 2 when an image is captured.

The capsule endoscope 10 has a built-in circuit board (not illustrated) on which a drive circuit or the like that drives each of the imaging unit 11 and the illumination unit 12 is formed, and the imaging unit 11 and the illumination unit 12 are fixed to this circuit board in a state in which their fields face outward from one end portion of the capsule endoscope 10.

The signal processing unit 13 controls each unit in the capsule endoscope 10, performs A/D conversion on the imaging signal output from the imaging unit 11 to generate digital image data, and further performs specified signal processing on the digital image data.

The memory 14 temporarily stores therein various operations executed by the signal processing unit 13 and the image data subjected to the signal processing in the signal processing unit 13.

The transmitting unit 15 and the antenna 16 superimpose, together with related information, the image data stored in the memory 14 on the wireless signal, and transmit them to outside.

The battery 17 supplies electric power to each unit in the capsule endoscope 10. The battery 17 includes a power source circuit that performs boosting or the like of electric power supplied from a primary battery or secondary battery, such as a button battery.

After being swallowed by the subject 2, the capsule endoscope 10 sequentially captures images of the biological sites (the esophagus, stomach, small intestine, large intestine, and the like) at specified time intervals (for example, 0.5 second intervals) while moving in the digestive tract of the subject 2 due to peristaltic movement and the like of the organs. The image data and related information generated by this image capturing operation are sequentially transmitted wirelessly to the receiving device 20. The related information includes identification information (for example, a serial number) or the like allocated in order to individually identify the capsule endoscope 10.

The receiving device 20 receives the image data and related information wirelessly transmitted from the capsule endoscope 10, via the receiving antenna unit 3, which has a plurality of (eight in FIG. 1) receiving antennas 3a to 3h. Each of the receiving antennas 3a to 3h is realized by using a loop antenna, for example, and the receiving antennas 3a to 3h are arranged at specified positions (for example, positions corresponding to respective organs in the subject 2 that are a passage route of the capsule endoscope 10) on an external surface of the body of the subject 2.

As illustrated in FIG. 2, the receiving device 20 includes: a receiving unit 21; a signal processing unit 22; a memory 23; a data transmitting and receiving unit 24; an operating unit 25; a display unit 26; a control unit 27 that controls each of these units; and a battery 28 that supplies electric power to each of these units.

The receiving unit 21 receives the image data wirelessly transmitted from the capsule endoscope 10, via the receiving antennas 3a to 3h.

The signal processing unit 22 performs specified signal processing on the image data received by the receiving unit 21.

The memory 23 includes: an image data storage unit 23a that stores therein the image data having been subjected to the signal processing in the signal processing unit 22 and their related information; en examination information storage unit 23b that stores therein examination information registered by the examination information management apparatus 30; and a server ID storage unit 23c that maintains a server ID. The examination information and the server ID will be described later.

The data transmitting and receiving unit 24 is an interface connectable to a USB or a communication line, such as a wire LAN or a wireless LAN, and under control of the control unit 27, receives various information, such as the examination information, from the examination information management apparatus 30 and transmits the image data and related information stored in the memory 23 to the examination information management apparatus 30.

The operating unit 25 is used when a user inputs various setting information or the like into the receiving device 20.

The display unit 26 displays information related to the examination (examination information, patient information, and the like) and various setting information or the like input by the user.

The receiving device 20 is attached to and carried by the subject 2 while image capturing by the capsule endoscope 10 is being carried out (for example, until the capsule endoscope 10 is excreted after being swallowed by the subject 2 and passing through the digestive tract). During that time period, the receiving device 20 further adds related information, such as received strength information and received time information for each of the receiving antennas 3a to 3h, to the image data received via the receiving antenna unit 3, and causes the image data storage unit 23a to store therein these image data and related information. After the image capturing by the capsule endoscope 10 is finished, the receiving device 20 is removed from the subject 2, connected now to the examination information management apparatus 30, and transfers (downloads) the image data and related information stored in the image data storage unit 23a to the examination information management apparatus 30. In FIG. 1, by connecting a cradle 20a to a USB port of the examination information management apparatus 30 and setting the receiving device 20 in the cradle 20a, the receiving device 20 is connected to the examination information management apparatus 30.

The examination information management apparatus 30 is configured by using, for example, a work station including a display device 30a, such as a CRT display or a liquid crystal display. The examination information management apparatus 30 initializes the receiving device 20 before the examination using the capsule endoscope 10, acquires, from the receiving device 20, the image data accumulated in the receiving device 20 in the examination, performs specified processing on the image data, and generates an image for observation used in medical diagnosis of the subject 2. The initialization herein refers to a process of deleting information that has been stored in the memory 23 of the receiving device 20 and registering, with the receiving device 20, examination information related to an examination to be carried out next. Further, this registration refers to a process of transmitting targeted information to the receiving device 20 and causing the information to be stored in a specified storage area of the memory 23.

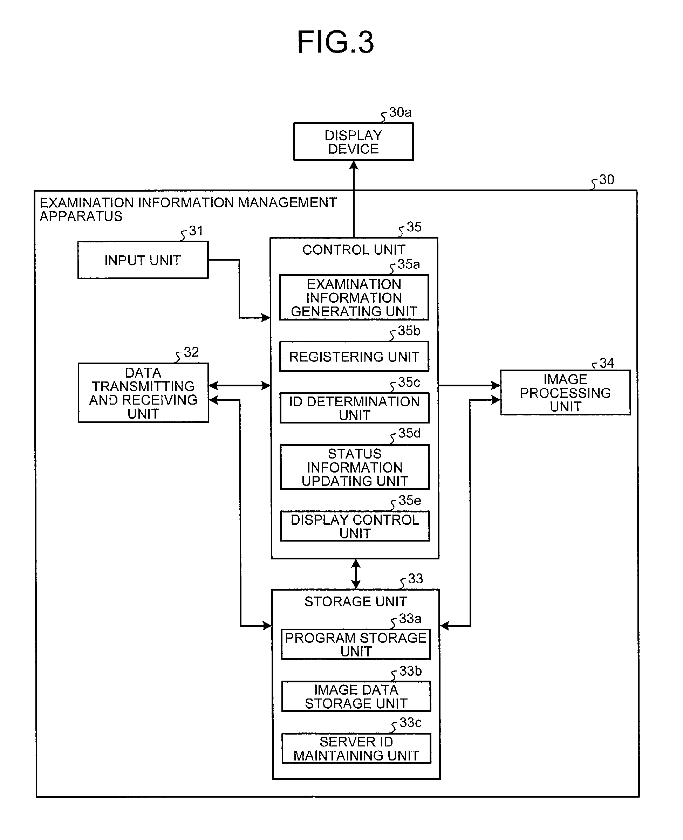

FIG. 3 is a block diagram illustrating a schematic configuration of the examination information management apparatus 30. As illustrated in FIG. 3, the examination information management apparatus 30 includes: an input unit 31; a data transmitting and receiving unit 32; a storage unit 33; an image processing unit 34; and a control unit 35 that comprehensively controls these units.

The input unit 31 is realized by an input device, such as a key board and a mouse, a touch panel, or various switches. The input unit 31 receives input of information and an instruction according to user's manipulation.

The data transmitting and receiving unit 32 is an interface connectable to a USB or a communication line, such as a wire LAN or wireless LAN, and includes a USB port and a LAN port. In this embodiment, the data transmitting and receiving unit 32: is connected to the receiving device 20 via the cradle 20a, which is connected to the USB port; is connected to the server 40 via the network N1; and performs transmission and reception of data to and from the receiving device 20 and the server 40.

The storage unit 33 is realized by: a semiconductor memory, such as a flash memory, a RAM, or a ROM; or a recording medium, such as an HDD, an MO, a CD-R, or a DVD-R, and a read and write device or the like that performs reading and writing of information from and to the recording medium. The storage unit 33 includes: a program storage unit 33a that stores therein a program and various information for causing the examination information management apparatus 30 to operate and execute various functions; an image data storage unit 33b that temporarily stores therein the image data and related information acquired via the receiving device 20; and a server ID maintaining unit 33c that maintains therein a server ID, which is unique identification information of the server 40 connected to the examination information management apparatus 30.

The image processing unit 34 is realized by hardware, such as a CPU, and by reading a specified program stored in the program storage unit 33a, performs specified image processing on the image data stored in the image data storage unit 33b and performs, based on the related information (received strength information and received time information) of the image data, a position detection process of detecting a position of the capsule endoscope during imaging of in-vivo images. In more detail, the image processing unit 34 generates image data for display by performing image processing, such as a white balance process, demosaicing, color conversion, density conversion (gamma conversion or the like), smoothing (noise reduction or the like), or sharpening (edge enhancement or the like), on the image data, and further, performs image processing for a specified diagnosis support, such as an average color calculation process, a lesion detection process, a red color detection process, or an organ detection process.

The control unit 35 is realized by hardware, such as a CPU, and by reading various programs stored in the storage unit 33, performs transfer or the like of instructions and data to respective units forming the examination information management apparatus 30 and comprehensively controls operations of the overall examination information management apparatus 30, based on a signal input via the input unit 31 or image data or the like input from the data transmitting and receiving unit 32.

In more detail, the control unit 35 includes an examination information generating unit 35a, a registering unit 35b, an ID determination unit 35c, a status information updating unit 35d, and a display control unit 35e. The examination information generating unit 35a generates examination information based on information input via the input unit 31 and causes the server 40 to store the examination information. Upon initialization of the receiving device 20, the registering unit 35b registers with the receiving device 20 examination information selected and a server ID maintained by the server ID maintaining unit 33c. When downloading image data from the receiving device 20, the ID determination unit 35c determines whether or not the server ID registered with the receiving device 20 matches the server ID maintained by the server ID maintaining unit 33c. The status information updating unit 35d updates, as required, examination status information included in examination information stored in a later described examination information database 42a, according to progress of an examination, such as "before initialization of the receiving device 20", "initialization of the receiving device 20 done", "examination in progress", "examination finished", "download of image data", "report generation", and the like. The display control unit 35e performs control of causing the display device 30a to display, in a specified format, various information input via the input unit 31 and an image and the like processed by the image processing unit 34.

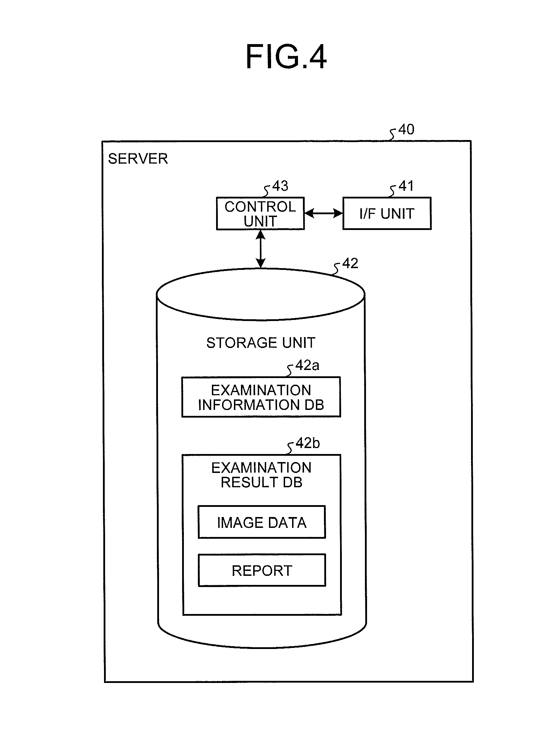

FIG. 4 is a block diagram illustrating a schematic configuration of the server 40. As illustrated in FIG. 4, the server 40 includes: an interface (I/F) unit 41 that is connectable with a communication line, such as a wire LAN or a wireless LAN; a storage unit 42; and a control unit 43 that comprehensively controls operations of the server 40, and the server 40 stores examination information and image data input from the examination information management apparatus 30.

The storage unit 42 includes an examination information database (DB) 42a and an examination result database (DB) 42b.

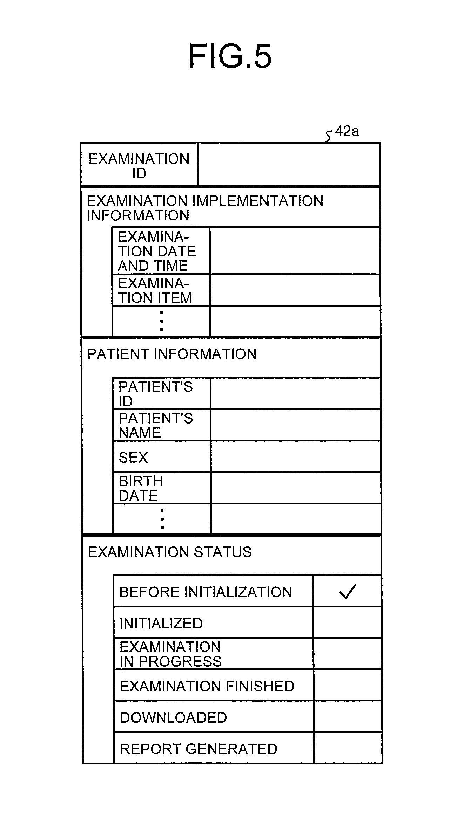

The examination information database 42a stores therein examination information generated by the examination information management apparatus 30. FIG. 5 is a diagram illustrating an example of contents of the examination information stored in the examination information database 42a. As illustrated in FIG. 5, the examination information includes: examination implementation information, such as an examination ID given uniquely to each examination, an examination date and time, an examination item, and the like; and patient information, such as a patient's ID, a patient's name, sex, birth date, and the like; and examination status information.

Of these, the examination status information includes examination statuses, such as "before initialization", "initialized", "examination in progress", "examination finished", "downloaded", "report generated", and the like. Of these, "before initialization" indicates that the examination information has not been registered with the receiving device 20 yet. "Initialized" indicates that the examination information has been registered with the receiving device 20. "Examination in progress" indicates that the capsule endoscope 10 has been introduced into the subject 2 and examination of capturing images of inside of the subject 2 is being carried out. "Examination finished" indicates that the examination has finished. "Downloaded" indicates that a download process, of transferring image data, which are wirelessly transmitted from the capsule endoscope 10 during an examination and accumulated in the receiving device 20, to the examination information management apparatus 30 and performing image processing and a position detection process, has finished. "Report generated" indicates that a report of a result of diagnosis on in-vivo images generated based on image data subjected to the image processing in the examination information management apparatus 30 has been generated. In FIG. 5, the examination status is "before initialization".

The examination result database 42b stores therein the image data subjected to the image processing in the examination information management apparatus 30 and the report generated in the examination information management apparatus 30. An examination ID is given to these image data and report, and via the examination ID, they are associated with the examination information.

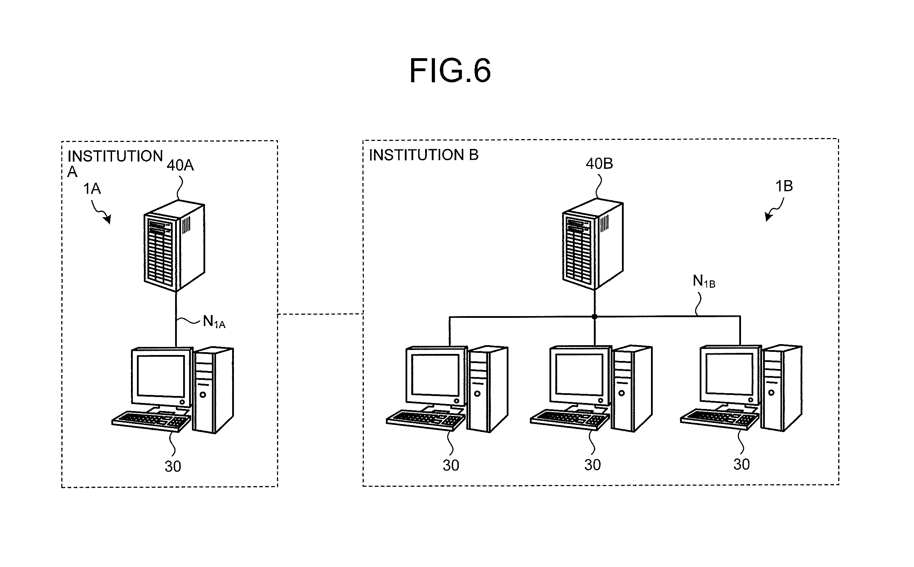

FIG. 6 is a schematic diagram illustrating an operation form of the examination information management system 1 illustrated in FIG. 1. As illustrated in FIG. 6, the examination information management system 1 is provided in each of institutions "A" and "B", which are different from each other. The institutions "A" and "B" are, for example, medical institutions, such as centers or the like that: receive receiving devices 20 used in examinations from a hospital that performs the examinations and diagnosis on patients or a hospital that performed the examinations; download image data stored in the receiving devices 20; and are specialized in performing observation operations of images based on the image data. Hereinafter, the system provided in the institution "A" will be referred to as an examination information management system 1A and the system provided in the institution "B" will be referred to as an examination information management system 1B. The examination information management systems 1A and 1B may be connected to each other via a public communication line, such as a telephone line or the Internet, or may not be connected.

In each of the examination information management systems 1A and 1B, one of servers 40A and 40B, to which different server IDs are given for the respective institutions, is provided. Further, to each of the servers 40A and 40B, one examination information management apparatus 30 or a plurality of examination information management apparatuses 30 is or are connected via a network in that institution. For example, to the server 40A, one examination information management apparatus 30 is connected via a network N_{1A}, and to the server 40B, three examination information management apparatuses 30 are connected via a network N_{1B}. Examination information and results of examination generated in the institutions "A" and "B" are collected and centrally managed by the servers 40A and 40B in the institutions A and B, respectively.

As described above, by building the examination information management systems 1A and 1B of similar arrangements respectively in the different institutions A and B, applications, such as role sharing of dedicatedly performing examinations in the one institution "A" and dedicatedly performing observations in the other institution "B", as well as sharing of portable devices such as the receiving devices 20 and the like, become possible.

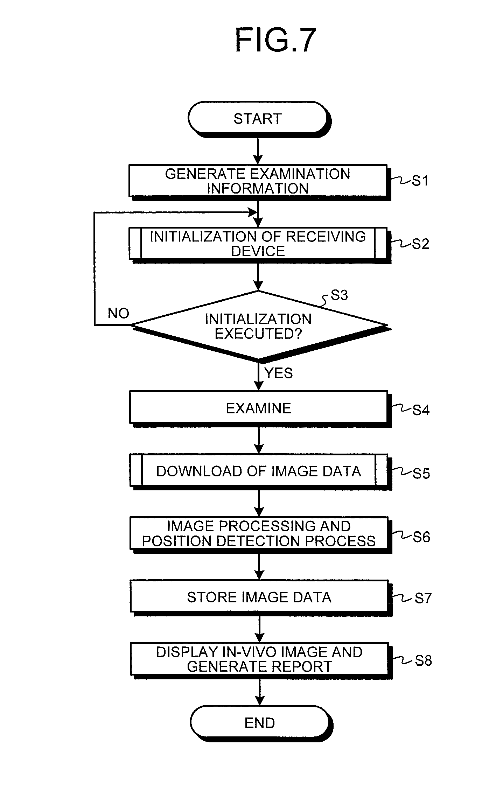

Next, operations of the examination information management system 1 will be described. FIG. 7 is a flow chart illustrating a series of processes related to an examination using the capsule endoscope 10.

First, at step S1, the examination information management apparatus 30 generates, based on an examination order input via the input unit 31, examination information. In more detail, when a user performs manipulation of inputting respective items of the examination information illustrated in FIG. 5 by using the input unit 31, the examination information generating unit 35a generates new examination information and causes the server 40 to store the new examination information. When the examination information is initially generated, the examination status is set to "before initialization".

At subsequent step S2, the examination information management apparatus 30 performs initialization of the receiving device 20. The initialization of the receiving device 20 is started by connecting the cradle 20a to the examination information management apparatus 30 and setting the receiving device 20 in the cradle 20a. A process of the initialization is performed before start of image capturing by the capsule endoscope 10.

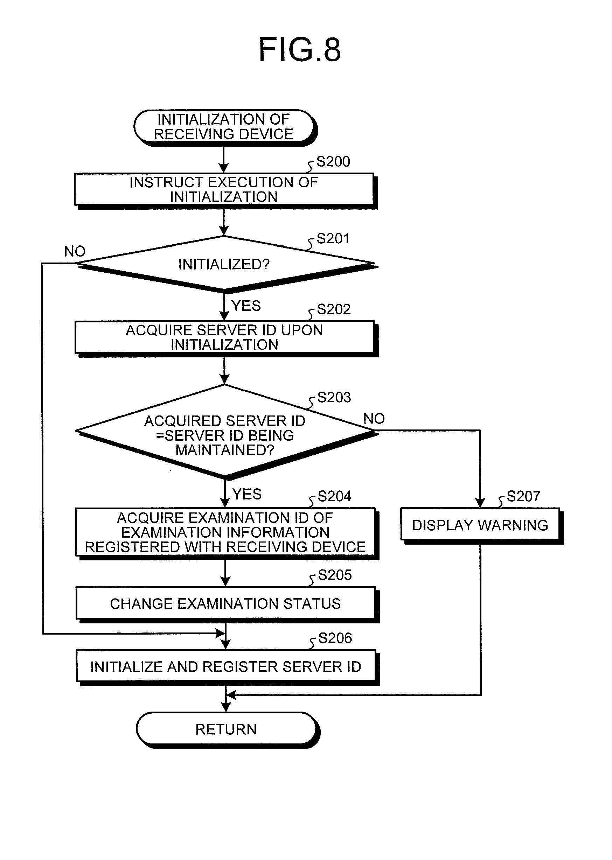

FIG. 8 is a flow chart illustrating an initialization process of the receiving device 20 executed by the examination information management apparatus 30. When the receiving device 20 is set in the cradle 20a, the examination information management apparatus 30 recognizes the receiving device 20.

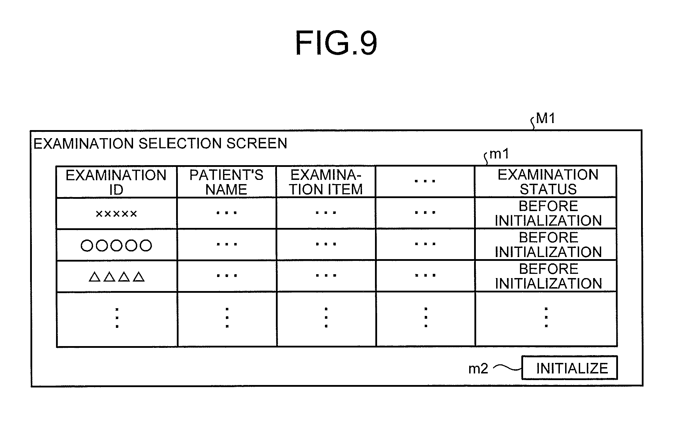

At step S200, the control unit 35 receives an instruction to execute the initialization. In more detail, the control unit 35 first extracts, from the examination information database 42a, examinations for which their examination statuses have been set to "before initialization" and causes the display device 30a to display an examination selection screen M1 as illustrated in FIG. 9, for example, in order to cause a user to select an examination to be registered. The examination selection screen M1 includes: an examination display area m1, where a list of examination information before initialization is displayed; and an initialization button m2. When the user specifies one examination displayed in the examination display area m1 by a specified pointer operation (for example, a click operation) using the input unit 31 and further presses the initialization button m2, the process proceeds to step S201.

At step S201, the control unit 35 determines whether or not the receiving device 20 has been initialized. Specifically, whether of not any examination information has been stored already in the examination information storage unit 23b of the receiving device 20 is checked.

If the receiving device 20 has not been initialized (step S201: No), the process proceeds to step S206, and the examination information management apparatus 30 performs the initialization of the receiving device 20 and registers a server ID with the receiving device 20. In more detail, the registering unit 35b acquires, from the examination information database 42a, examination information related to the selected examination and transmits the examination information to the receiving device 20, according to a selection signal input via the input unit 31. Further, the registering unit 35b also transmits the server ID, which is maintained by the server ID maintaining unit 33c, to the receiving device 20. Accordingly, the receiving device 20 causes the examination information storage unit 23b to store therein the received examination information and causes the server ID storage unit 23c to store the server ID.

Further, when that is done, the status information updating unit 35d changes the examination status of that examination information from "before initialization" to "initialized" in the examination information database 42a.

On the contrary, if the receiving device 20 has been initialized already (step S201: Yes), the examination information management apparatus 30 acquires the server ID stored in the server ID storage unit 23c from the receiving device 20 (step S202).

At subsequent step S203, the ID determination unit 35c determines whether or not the server ID acquired from the receiving device 20 matches the server ID maintained by the server ID maintaining unit 33c. That is, whether or not the server 40 (examination information database 42a) that acquired the examination information when the receiving device 20 was initialized matches the server 40 (same as above) that acquires the examination information upon the current initialization, is determined.

If these server IDs match each other (step S203: Yes), the examination information management apparatus 30 acquires an examination ID of the examination information registered with the receiving device 20 (step S204).

At subsequent step S205, the status information updating unit 35d searches through the examination information database 42a based on the examination ID acquired from the receiving device 20 and changes the examination status of the corresponding examination information. That is, the examination status "initialized" is canceled and returned to "before initialization".

Thereafter, the process proceeds to step S206, initialization on the receiving device 20 is performed, and the examination status of the corresponding examination information in the examination information database 42a is changed from "before initialization" to "initialized".

On the contrary, if, at step S203, the server IDs do not match each other (step S203: No), the examination information management apparatus 30 causes the display device 30a to display a message indicating a warning that the server IDs do not match (step S207). In that case, the initialization of the receiving device 20 is not performed and the process returns to a main routine.

At step S3 subsequent to step S2, if the initialization of the receiving device 20 has been executed (step S3: Yes), the process proceeds to step S4 next. On the contrary, if the initialization of the receiving device 20 has not been executed (step S3: No), the process returns to step S2 again, after preparing another receiving device 20, for example.

At step S4, an examination using the capsule endoscope 10 is performed on the subject 2. Specifically, the receiving antenna unit 3 is attached to the subject 2, the initialized receiving device 20 is caused to be carried by the subject 2, and the capsule endoscope 10 is caused to be swallowed by the subject 2. Thereby, the capsule endoscope 10 performs image capturing while moving inside the subject 2 and sequentially transmits image data to the receiving device 20.

When the reception of the image data from the capsule endoscope 10 in the receiving device 20 is finished, the user removes the receiving device 20 from the subject 2, and performs downloading of the image data (step S5). The downloading of the image data is started by setting the receiving device 20 in the cradle 20a connected to the examination information management apparatus 30.

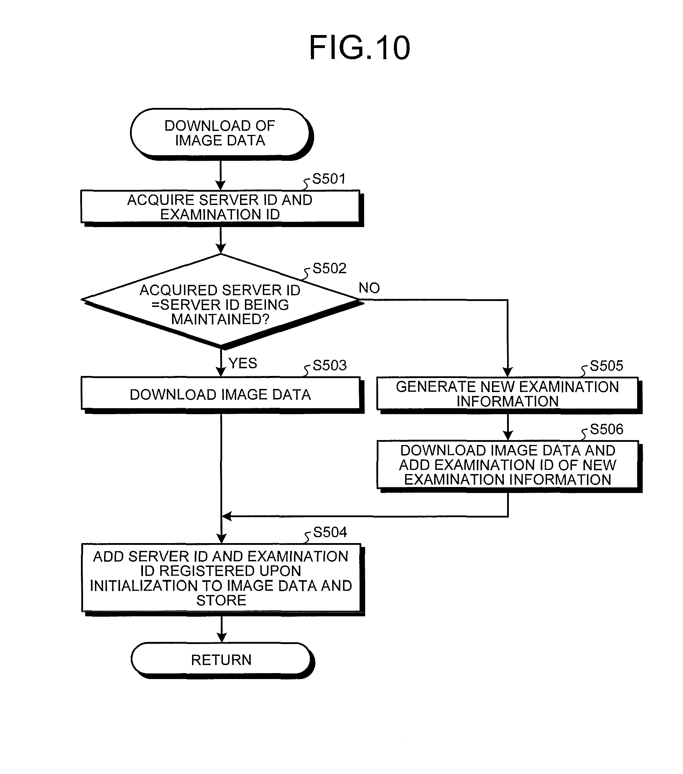

FIG. 10 is a flow chart illustrating a download process of image data executed by the examination information management apparatus 30. The control unit 35 acquires the server ID and examination ID stored in the server ID storage unit 23c from the receiving device 20 at step S501, when the control unit 35 recognizes the receiving device 20.

At subsequent step S502, the ID determination unit 35c determines whether or not the server ID acquired from the receiving device 20 matches the server ID maintained by the server ID maintaining unit 33c. That is, whether or not the server 40 (examination information database 42a) that acquired the examination information when the receiving device 20 was initialized matches the server 40 (same as above) that stores the current examination result.

If these server IDs match each other (step S502: Yes), the control unit 35 executes downloading of image data from the receiving device 20 (step S503).

At subsequent step S504, the control unit 35 adds the server ID and examination ID registered when the receiving device 20 was initialized to the downloaded image data and causes the image data storage unit 33b to store them therein.

On the contrary, if the server IDs do not match each other at step S502 (step S502: No), the examination information generating unit 35a generates, based on the examination information registered with the receiving device 20, new examination information (step S505). In more detail, the examination information generating unit 35a takes in the examination information stored in the examination information storage unit 23b from the receiving device 20 and stores the examination information as new examination information in the examination information database 42a of the server 40. Or, the examination information generating unit 35a may generate new examination information based on information input by the user using the input unit 31 and store the new examination information in the examination information database 42a. In that case, the user refers to the examination information displayed by the display unit 26 of the receiving device 20 and performs the input operation on the input unit 31.

At subsequent step S506, the control unit 35 downloads image data from the receiving device 20, adds an examination ID of the newly generated examination information to the image data for association with the examination information.

Thereafter, the process proceeds to step S504, and the control unit 35 further gives, to the downloaded image data and the newly generated examination information, the server ID and examination ID registered when the receiving device 20 was initialized and causes the image data storage unit 33b to store them therein.

Thereafter, the process returns to the main routine.

At step S6 subsequent to step S5, the image processing unit 34 performs specified image processing and a position detection process on the image data stored in the image data storage unit 33b.

Furthermore, at step S7, the control unit 35 stores the image processed image data in the server 40 (examination result database 42b).

Thereafter, the user, as required, performs observation by causing the display device 30a to display images (in-vivo images) based on the image data stored in the server 40 and generates a report including findings (step S8). The generated report is stored in the examination result database 42b in association with the examination information.

Accordingly, the series of processes related to the examination using the capsule endoscope 10 end.

As described above, according to the embodiment, since the examination information in the server 40 is not deleted even after the initialization of the receiving device 20, and the examination information and the examination statuses are centrally managed in the server 40 via the examination information management apparatus 30, relatedness is able to be given to the examination information related to a plurality of examinations (examinations performed on the same patient, which are performed at different times and are of different types).

Further, according to the embodiment, whether or not the image data downloaded from the receiving device 20 are related to the examination information managed by that examination information management system 1 is determined, and if they are related to the examination information managed by that examination information management system 1, the downloaded image data are associated with the existing examination information. On the contrary, if there is no relation to the examination information managed by the examination information management system 1, after generating new examination information, the downloaded data are associated with the new examination information. Therefore, as illustrated in FIG. 6, for example, even if the institution that has performed the initialization of the receiving device 20 (for example, the institution "A") and the institution that performs downloading from the receiving device 20 (for example, the institution "B") are different from each other, downloading is able to be performed and management by associating the image data with the appropriate examination information becomes possible.

Further, in the embodiment, if the image data not related to the examination information managed by that examination information management system 1 are downloaded, the image data are associated with new examination information and the server ID and examination ID registered with the receiving device 20 originally are given to the new examination information and the image data. Thereby, the downloaded image data are able to be easily associated with the original examination information when the downloaded image data are transferred to the original examination information management system 1 (that has performed the initialization of the receiving device 20). Therefore, as illustrated in FIG. 6, for example, a flexible application is possible, like performing a capsule endoscope examination by using the receiving device 20 initialized in the institution "A" and thereafter performing downloading and image processing of image data by carrying the receiving device 20 used in the examination into the institution "B", a doctor belonging to the institution "B" generating a report, and transferring the image data and the report to the institution "A".

Further, in the embodiment, if initialization (that is, registration of examination information by overwriting) is performed further on the receiving device 20 that has been initialized, by comparing the server ID registered with the receiving device 20 and a unique server ID of the examination information management system 1 with each other, whether or not the examination information registered with the receiving device 20 is the examination information managed by that examination information management system 1 is determined. If it is the examination information managed by that examination information management system 1, after the examination status of that examination information is changed in the server 40, initialization of the receiving device 20 is permitted. On the contrary, if it is not the examination information managed by that examination information management system 1, further initialization of the receiving device 20 is prohibited.

Specifically, as illustrated in FIG. 6, if the receiving device 20 initialized in the institution "A" is further initialized in the same institution "A", after the examination status of the initialized examination information stored in the examination information database 42a is changed to "before initialization", initialization of the receiving device 20 with other examination information is performed. If the receiving device 20 initialized in the institution "A" is lent out to the institution "B" and is attempted to be initialized in the institution "B", the examination information stored in the receiving device 20 is not a target to be managed by the institution "B" and thus that initialization is prohibited. Accordingly, even if lending and borrowing the receiving device 20 between the institutions "A" and "B" are to be performed, circumstances, such as deleting the examination information registered with the receiving device 20 by mistake, and as a result, the examination status on the system and the actual examination status being deviated from each other, are able to be prevented.

### (First Modified Example)

Next, a first modified example of the embodiment of the present invention will be described.

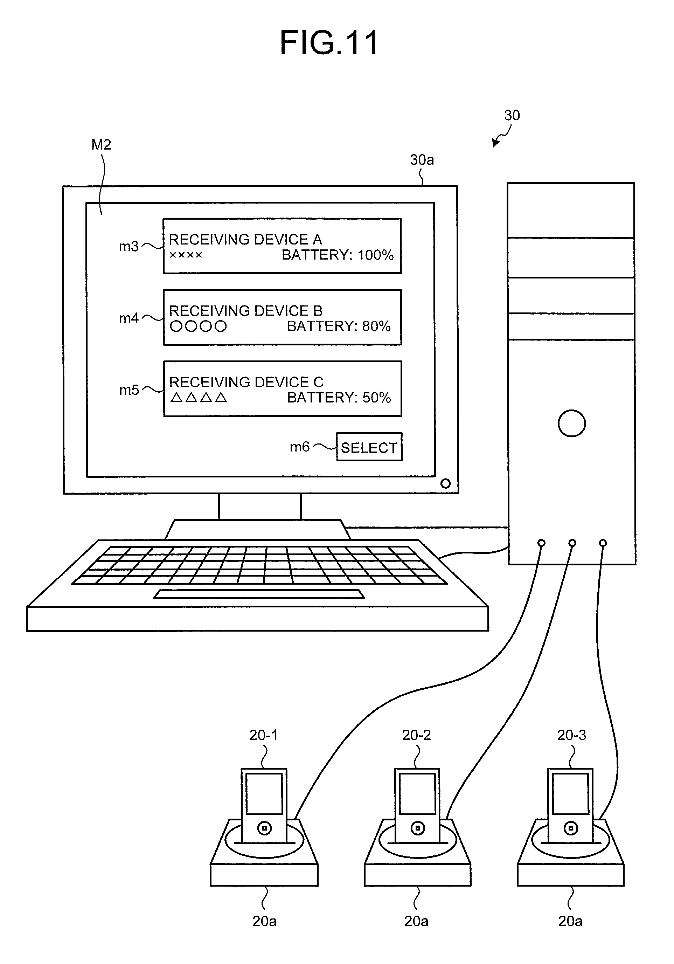

FIG. 11 is a schematic diagram illustrating a part of an examination information management system according to the first modified example. In the examination information management system illustrated in FIG. 11, with respect to one examination information management apparatus 30, a plurality of (three in FIG. 11) receiving devices 20-1, 20-2, and 20-3 are set via cradles 20a. Hereinafter, an initialization process of these receiving devices 20-1 to 20-3 will be described. Operations of the whole examination information management system according to the first example are similar to those illustrated in FIG. 7, and a detailed process executed by the examination information management apparatus 30 in step S2 is different from that of the above described embodiment.

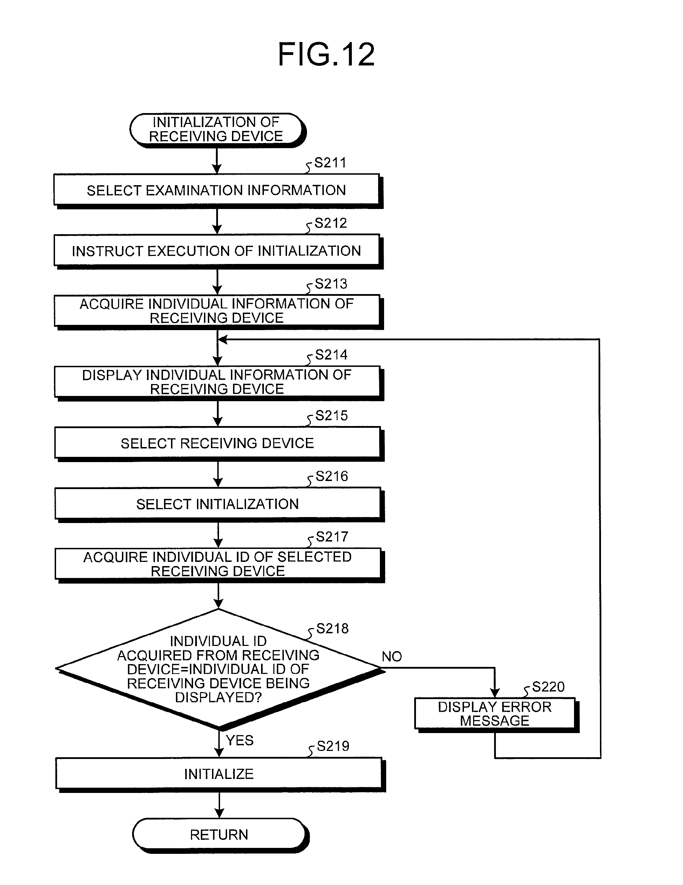

FIG. 12 is a flow chart illustrating a process executed by the examination information management apparatus 30 according to the first modified example.

After recognizing the receiving devices 20-1 to 20-3, the examination information management apparatus 30 causes a user to select examination information to be registered when the receiving devices 20-1 to 20-3 are initialized (step S211). In order to do so, the control unit 35 first causes the display device 30a to display the examination selection screen M1 illustrated in FIG. 9, for example. The user is able to select a desired examination by a specified pointer operation using the input unit 31 on the examination selection screen M1. The control unit 35 determines the selected examination according to a selection signal input via the input unit 31.

At subsequent step S212, the control unit 35 receives an instruction to execute initialization for the selected examination information according to a signal (for example, a signal input according to a pointer operation on the initialization button m2) input via the input unit 31.

At step S213, the examination information management apparatus 30 acquires individual information from each of receiving devices 20-1 to 20-3. The individual information includes, for example, an individual ID uniquely given to each of the receiving devices 20-1 to 20-3, a model, a specification, a battery charging state, and the like.

At step S214, the display control unit 35e causes the display device 30a to display a list of the acquired individual information. A screen M2 illustrated in FIG. 11 illustrates an example of a display screen of the individual information of the receiving devices 20-1 to 20-3. On this display screen M2, in addition to display columns m3 to m5 of the individual information of the receiving devices 20-1 to 20-3, a selection button m6 is provided.

At step S215, the control unit 35 causes a user to select a receiving device to be initialized, from the receiving devices 20-1 to 20-3. The user is able to select a desired receiving device by a pointer operation on any of the display columns m3 to m5, for example. The control unit 35 determines the selected receiving device according to a selection signal input via the input unit 31.

At subsequent step S216, according to the signal input via the input unit 31 (for example, a signal input according to a pointer operation on the selection button m6), the control unit 35 selects initialization of the selected receiving device.

At subsequent step S217, the control unit 35 acquires an individual ID from the selected receiving device.

At subsequent step S218, the ID determination unit 35c determines whether or not the individual ID of the receiving device acquired in step S217 matches the individual ID of the selected receiving device being displayed on the screen M2.

If these individual IDs match each other (step S218: Yes), the control unit 35 executes initialization of the selected receiving device (step S219). That is, the examination information of the examination selected in step S211 and the server ID maintained by the server ID maintaining unit 33c are registered with the receiving device. Thereafter, the process returns to the main routine.

On the contrary, if the individual IDs do not match each other (step S218: No), the display control unit 35e causes the display device 30a to display an error message (step S220). Thereafter, initialization of the selected receiving device is not executed and the process returns to step S214.

After the individual information of the receiving devices 20-1 to 20-3 is displayed on the screen M2 (step S214), the user may check the individual information and change the receiving devices 20-1 to 20-3 set in the cradles 20a. For example, if a receiving device, which has a charging amount that is not sufficient, or a receiving device, which has not been planned to be used, has been set by mistake, such a situation is generated.

In that case, when initialization is selected (step S216), there is a possibility that the receiving device selected on the screen M2 by the user and the receiving device that is actually being the target of initialization do not match each other. Therefore, in the first modified example, by comparing the individual IDs of the receiving devices before executing the initialization, initialization of a receiving device not desired by the user is able to be prevented.

### (Second Modified Example)

Next, a second modified example of the embodiment of the present invention will be described.

In the above described embodiment, if the server ID acquired from the receiving device 20 does not match the server ID maintained by the server ID maintaining unit 33c (step S203 of FIG. 8: No), initialization of the receiving device 20 is terminated. In this second modified example, an example, in which even if the two IDs do not match each other, initialization of the receiving device 20 is able to be continued, will be described.

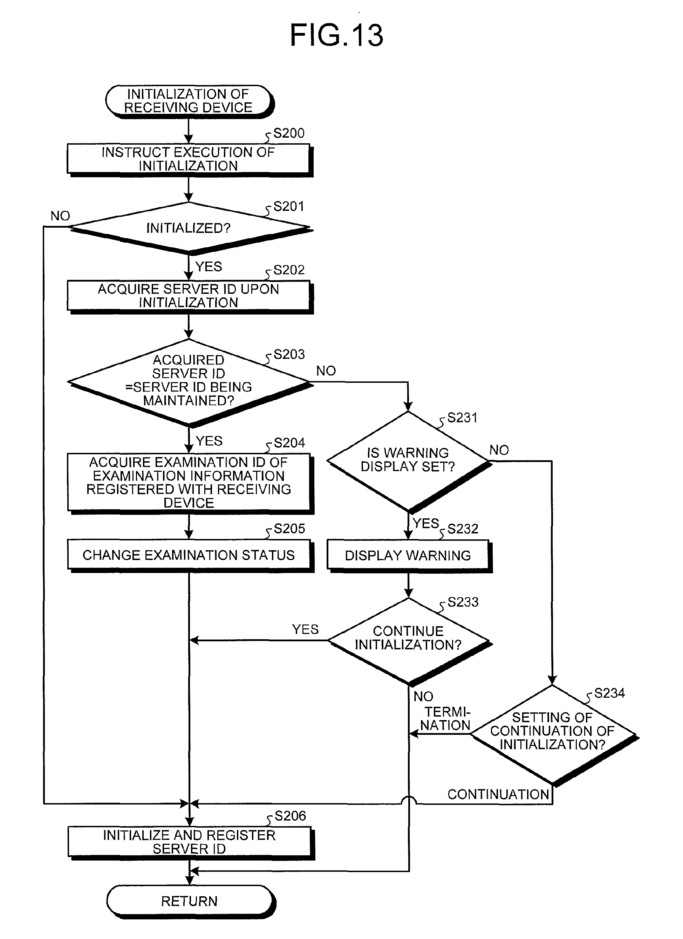

FIG. 13 is a flow chart illustrating an initialization process of the receiving device 20 executed by the examination information management apparatus 30 according to the second modified example. Steps S200 to S206 illustrated in FIG. 13 correspond to those of the above described embodiment.

At step S203, if the two server IDs do not match each other (step S203: No), the control unit 35 checks presence or absence of warning display setting (step S231). This warning display refers to an operation of causing the display device 30a to display a message that the IDs do not match each other for a user if the two server IDs do not match each other. Whether or not to perform the warning display is set by the user beforehand.

If the warning display setting has been performed (step S231: Yes), the display control unit 35e causes the display device 30a to display the message that the two server IDs do not match each other (step S232).

At subsequent step S233, the control unit 35 checks with the user whether or not to continue the initialization. Specifically, a confirmation screen, which includes: a message, such as "Do you want to continue initialization?"; a confirmation button that is able to be selected by a pointer operation using the input unit 31; and the like, is caused to be displayed by the display device 30a.

If the continuation of the initialization has been confirmed (for example, if the confirmation button has been selected, step S233: Yes), the process proceeds to step S206.

On the contrary, if the continuation of the initialization is not confirmed (for example, if the confirmation button is not selected, step S233: No), the process returns to the main routine without execution of the initialization of the receiving device 20.

Further, at step S231, if the warning display setting has not been performed (step S231: No), the control unit 35 checks contents of the setting of the continuation of the initialization (step S234). The contents of the setting of the continuation of the initialization have two types, which are continuing the initialization without checking with the user and terminating the initialization without checking with the user, if the two server IDs do not match each other, and are set beforehand by the user.

If the setting to continue the initialization has been performed (step S234: continuation), the process proceeds to step S206. If the setting to terminate the initialization has been performed (step S234: termination), the initialization of the receiving device 20 is not executed and the process returns to the main routine.

In the above described embodiment of the present invention and the modified examples thereof, the server 40 is caused to store the examination information database 42a and the examination result database 42b, but instead of the server 40, they may be stored in the storage unit 33 in the examination information management apparatus 30. In this case, even if a plurality of examination information management apparatuses 30 are provided in one examination information management system, only one of them is specified as the examination information management apparatus to store therein the examination information database 42a and examination result database 42b. Then, a unique identification ID of the specified examination information management apparatus may be used as a database identification ID common in that examination information management system.

The invention is not limited to the embodiments and modified examples thereof, and various inventions may be formed by appropriately combining a plurality of elements disclosed in the respective embodiments and modified examples. For example, some elements may be excluded from the whole elements illustrated in the embodiments and modified examples, or the elements illustrated in the different embodiments and modified examples may be appropriately combined.

### Reference Signs List

- 1, 1A, 1B: examination information management system
- 2: subject
- 3: receiving antenna unit
- 3a to 3h: receiving antenna
- 10: capsule endoscope
- 11: imaging unit
- 12: illumination unit
- 13: signal processing unit
- 14: memory
- 15: transmitting unit
- 16: antenna
- 17: battery
- 20: receiving device
- 20a: cradle
- 21: receiving unit
- 22: signal processing unit
- 23: memory
- 23a: image data storage unit
- 23b: examination information storage unit
- 23c: server ID storage unit
- 24: data transmitting and receiving unit
- 25: operating unit
- 26: display unit
- 27: control unit
- 28: battery
- 30: examination information management apparatus
- 30a: display device
- 31: input unit
- 32: data transmitting and receiving unit
- 33: storage unit
- 33a: program storage unit
- 33b: image data storage unit
- 33c: server ID maintaining unit
- 34: image processing unit
- 35: control unit
- 35a: examination information generating unit
- 35b: registering unit
- 35c: ID determination unit
- 35d: status information updating unit
- 35e: display control unit
- 40, 40A, 40B: server
- 41: interface (I/F) unit
- 42: storage unit
- 42a: examination information database
- 42b: examination result database
- 43: control unit

## Claims

1. An examination information management apparatus that manages examination information in an examination using a capsule endoscope for capturing in-vivo images, and a receiving device for receiving image data acquired by the capsule endoscope through the capturing, the examination information management apparatus comprising:
a data transmitting and receiving unit that performs transmission and reception of data to and from the receiving device;
a storage unit that stores the examination information and the image data acquired in the examination;
an ID maintaining unit that maintains an identification ID for identifying the storage unit;
a registering unit that registers the examination information and the identification ID with the receiving device;
a determination unit that determines, when the image data that the receiving device has received from the capsule endoscope, the examination information and the identification ID are received from the receiving device via the data transmitting and receiving unit, whether or not the identification ID registered with the receiving device matches the identification ID maintained by the ID maintaining unit; and
an examination information generating unit that newly generates the examination information and causes the storage unit to store the image data acquired by the receiving device in association with the newly generated examination information, if the identification IDs are not determined by the determination unit to match each other.

2. The examination information management apparatus according to claim 1, wherein the examination information generating unit associates the identification ID registered with the receiving device with the examination information when the examination information generating unit causes the storage unit to store the newly generated examination information.

3. The examination information management apparatus according to claim 1, further comprising a status information updating unit that adds, to the examination information stored in the storage unit, information indicating that the examination information has been registered, when the registering unit registers the examination information and the identification ID with the receiving device, wherein
the determination unit determines whether or not the identification ID registered with the receiving device and the identification ID maintained by the ID maintaining unit match each other, when a process of registering, with the receiving device with which first examination information has been registered, second examination information different from the first examination information, is performed, and
if the identification IDs have been determined by the determination unit to match each other, the status information updating unit acquires the first examination information from the receiving device with which the first examination information has been registered, cancels, from the first examination information stored in the storage unit, information indicating that the first examination information has been registered, and adds, to the second examination information stored in the storage unit, information indicating that the second examination information has been registered.

4. An examination information management system, comprising:
the examination information management apparatus according to claim 1; and
the receiving device that receives the image data generated by the capsule endoscope.
